# EUROPEAN PATENT APPLICATION

(11) **EP 4 505 948 A1**
(43) Date of publication of application: **12.02.2025**
(21) Application number: 23189962.6
(22) Date of filing: 07.08.2023
(51) Int. Cl.: A61B 17/12

(54) **LEFT ATRIAL APPENDAGE IMPLANT**

(71) Applicant: Helmholtz-Zentrum hereon GmbH, 21502 Geesthacht (DE); Charité - Universitätsmedizin Berlin, 10117 Berlin (DE)
(72) Inventor: Großhauser, Johannes, 14513 Teltow (DE); Reinthaler, Markus, 12203 Berlin (DE); Polak-Krasna, Katarzyna, 12524 Berlin (DE); Landmesser, Ulf, 12205 Berlin (DE); Schröder, Mark, 12279 Berlin (DE)
(74) Representative: Uexküll & Stolberg

(57) **Abstract**

An implant (1) for occluding a left atrial appendage comprises a radially expansible element (2) configured to shift between a collapsed configuration and an expanded configuration. The radially expansible element (2) comprises a pair of core elements (3) which extends in direction of a longitudinal axis (4). The pair of core elements (3) comprises a first core element (5) and a second core element (6). The first and second core elements (5, 6) are stacked in direction of said longitudinal axis (4). The pair of core elements (3) has an outer circumference (7) and the first core element (5) and the second core element (6) each enclose a cavity (8). The first core element (5) and the second core element (6) are rotatable relative to one another.

The radially expansible element (2) further comprises a driving and locking element arranged in the respective cavities (8) of the pair of core elements (3) for rotating the first core element (5) and the second core element (6) relative to one another so as to move the radially expansible element (2) from the collapsed configuration into the expanded configuration and for locking the first core element (5) and the second core element (6) relative to one another, wherein the driving and locking element is adapted for being engaged with a rotatable pin mounted at the tip of an elongated catheter member.

The radially expansible element (2) further comprises a plurality of pairs of bendable spokes (9) extending from the pair of core elements (3) in an outward direction, each of the pairs of bendable spokes (9) having a first spoke member (10) and a second spoke member (11), the first and second spoke members (10, 11) being stacked in direction of said longitudinal axis (4), and each of said first and second spoke members (10, 11) having an inner end (12) and an outer end (13).

The radially expansible element (2) further comprises a plurality of shell elements (14) attached to the outer end (13) of the spoke members (9), and extending transversely from the outer end (13) of the spoke members (9) in a curved manner, the shell elements (14) having a proximal end (15) and a distal end (16).

The inner end (12) of the first spoke member (9) is connected to the outer circumference (7) of the first core element (5), and the inner end (12) of the second spoke member (6) is connected to the outer circumference (7) of the second core element (6). The outer ends (13) of the first and second spoke members (10, 11) are both connected to the proximal end (15) of the shell elements (14). The distal end (16) of the shell elements (14) is configured to overlap with a proximal end (15) of the shell elements (14) in the expanded configuration of the radially expansible element (2) so as to form a substantially circular outer circumference (17) of the radially expansible element (2).

## Description

### TECHNICAL FIELD

The present disclosure relates generally to medical devices and more preferably to medical devices that are adapted for use in percutaneous medical procedures such as implantation into the left atrial appendage (LAA) of a heart.

### BACKGROUND

Left atrial appendage occlusion (LAAO), also referred to as left atrial appendage closure (LAAC), is a treatment performed in high-risk patients for the prevention of thrombus formation in atrial fibrillation (AF) and for reducing the associated risk of stroke.

The left atrial appendage is a small organ attached to the left atrium of the heart. During normal heart function, as the left atrium constricts and forces blood into the left ventricle, the left atrial appendage constricts and forces blood into the left atrium. Patients suffering from atrial fibrillation, the left atrial appendage may not properly contract or empty, causing stagnant blood to pool within its interior, which can lead to the undesirable formation of thrombi within the left atrial appendage. Thrombi forming in the left atrial appendage may break loose from this area and enter the blood stream. Thrombi that migrate through the blood vessels may eventually plug a smaller vessel downstream and thereby contribute to stroke or heart attack. Clinical studies have shown that the majority of blood clots in patients with atrial fibrillation originate in the left atrial appendage.

In the last 20 years, atrial fibrillation has become one of the most important public health problems and a significant cause of increasing health care costs in western countries. In these countries, subjects aged 60 to 70 years show an AF incidence of 3.7% to 4.2% which may increase up to 11% to 17% in those aged 80 years and older.

As stated, atrial fibrillation is associated with an increased risk of thromboembolic events, including stroke, which is the reason why patients are usually started on oral anticoagulation therapy. However, despite a broad range of anticoagulant options and improved uptake in anticoagulation over the past decade, there are limitations to this approach. First, there is a significant proportion of high-risk patients who have haematological disorders, frailty or previous major bleeding, whose high risk of bleeding precludes the use of anticoagulants. Second, there are patients who may continue to suffer thromboembolic events despite receiving appropriate guideline-directed anticoagulation therapy. Third, anticoagulation therapy can significantly decrease the quality of life in patients, who are prone to bruising and bleeding. Furthermore, compliance and adherence with drug therapy may be suboptimal; discontinuation rates in randomized controlled trials (RCTs) of direct oral anticoagulants (DOACs) are between 21% and 27%.

As an alternative treatment for patients suffering from atrial fibrillation, medical devices have been developed which are delivered to the site of implantation via a delivery catheter and then deployed to close the left atrial appendage.

U.S. Patent 6,162,154 discloses a device, referred-to as the "Plaato" device, having an occluding member and a retention member secured to the occluding member. The retention member is configured to engage and attach to a surface of a body cavity and maintain a position of the occluding member to sealingly engage the inside surface of the body cavity and prevent the passage of embolic material or the like therethrough. The occluding member is a frame structure, typically of NiTi alloy. The frame structure has a barrier or mesh material disposed over it and preferably secured to it to act as a barrier to the passage of embolic material or fluids. The frame structure serves to support the barrier or mesh material in an outwardly expanded state to substantially occupy at least a portion of the cross section of a body cavity or passageway within which it is disposed.

U.S. Patent 6,6810,160 discloses another device, referred-to as the "Watchman" device, which lies wholly within the left atrial appendage and includes a membrane that spans the opening or ostium of the left atrial appendage. The Watchman device is typically porous. Additionally, the Watchman device uses a series of barbs or prongs which enter the tissue surrounding the ostium of the left atrial appendage to help secure the device during the acute phase of implant.

WO 2018/185255 A1 discloses a still further device for occlusion of the left atrial appendage comprising an implantable occlusion apparatus operably and detachably attached to an elongated catheter member configured for transluminal delivery and deployment of the occlusion apparatus in the body lumen. The occlusion apparatus comprises a radially expansible element that is adjustable between a contracted orientation suitable for transluminal delivery and a deployed orientation configured to occlude the body lumen.

US 2021/00164101 A1 discloses another device for occlusion of the left atrial appendage which comprises an expandable framework configured to shift between a collapsed configuration and an expanded configuration, wherein the expandable framework includes an attachment point configured to secure the expandable framework to a delivery device; and an occlusive element disposed on a proximal portion of the expandable framework, wherein the occlusive element covers the attachment point.

US 2021/02041060 A1 discloses yet another device for occlusion of the left atrial appendage which comprises a contact member configured to move between a first state and a second state, and a securing element, wherein the contact member is configured to move from the first state to the second state so that at least a portion of the contact member engages a wall portion of the LAA after the contact member has been advanced into the LAA, the contact member is configured to rotate at least in a first direction from a first rotational position to a second rotational position.

The devices are usually delivered to the site of implantation percutaneously, whereby a delivery catheter comprising an outer sheath housing a compressed or collapsed implant is advanced from the femoral vein into the heart and into the left atrial appendage, and unfolded at the site of implantation. Current catheter diameters for the LAA device delivery are in the range of 11 to 16 Fr, which corresponds to 3 to 5 mm. The diameter range for the sealing area of the LAA is from 17 to 32 mm (Watchman FLX Device).

In case the device is in the wrong position, it needs to be pulled back into the outer sheath and a new position needs to be found. If a suitable position cannot be found, then the device needs to be removed altogether via the outer sheath. To this end, it is necessary that the device can be removed from the LAA and pulled back into the outer sheath completely. It is also necessary for an anchoring system, which holds the device in place once implanted, to be folded before repositioning and retrieval no to damage the surrounding tissue.

Most of the existing devices do not allow a controlled deployment. That is, when the devices are pushed out of the outer sheath of the delivery catheter at the site of implantation, they immediately unfold in a manner similar to an umbrella. This makes it challenging to predict in which final position the device will unfold as regard the anchoring devices making a controlled fixation or repositioning difficult. Thus, there is a need for medical devices that are adapted for use in percutaneous medical procedures which can be expanded and positioned at the implantation site in a controlled manner.

Furthermore, the necessary diameter of a device for the occlusion of the LAA depends on the anatomy of the patient's LAA. Commercially available devices offer about 5 to 11 different sizes to suit the human anatomy. Thus, there is also a need for medical devices that are adapted for use in percutaneous medical procedures which can be adapted in its size so that the number of device sizes the clinician has to choose from is reduced.

It is an object of the invention to overcome at least one of the above-referenced problems.

### SUMMARY OF INVENTION

The objects are overcome by the provision of a device as defined in claim 1 and a system as defined in claim 12. Preferred embodiments are described in the appending claims.

The disclosure provides a device adapted for use in percutaneous medical procedures, in particular an implant for occluding a left atrial appendage and heart valve replacement devices, comprising a radially expansible element configured to shift between a collapsed configuration and an expanded configuration, wherein the radially expansible element comprises a pair of core elements which extends in direction of a longitudinal axis, wherein the pair of core elements comprises a first core element and a second core element, the first and second core elements being stacked in direction of said longitudinal axis, the pair of core elements having an outer circumference and the first core element and the second core element each enclosing a cavity, wherein the first core element and the second core element are rotatable relative to one another.

The radially expansible element further comprises a driving and locking element arranged in the respective cavities of the pair of core elements for rotating the first core element and the second core element relative to one another so as to move the radially expansible element from the collapsed configuration into the expanded configuration and for locking the first core element and the second core element relative to one another, wherein the driving and locking element is adapted for being engaged with a rotatable pin mounted at the tip of an elongated catheter member.

The radially expansible element further comprises a plurality of pairs of bendable spokes extending from the pair of core elements in an outward direction, each of the pairs of bendable spokes having a first spoke member and a second spoke member, the first and second spoke members being stacked in direction of said longitudinal axis, and each of said first and second spoke members having an inner end and an outer end.

The radially expansible element further comprises a plurality of shell elements attached to the outer end of the spoke members, and extending transversely from the outer end of the spoke members in a curved manner, the shell elements having a proximal end and a distal end.

The inner end of the first spoke member is connected to the outer circumference of the first core element, and the inner end of the second spoke member is connected to the outer circumference of the second core element. The outer ends of the first and second spoke members are both connected to the proximal end of the shell elements. The distal end of the shell elements is configured to overlap with a proximal end of the shell elements in the expanded configuration of the radially expansible element so as to form a substantially circular outer circumference of the radially expansible element.

Starting from the collapsed configuration of the radially expansible element the speed and the extent of the radial expansion of the expansible element can be controlled. The expansible element may be brought in the collapsed configuration by rotating the first and second core elements relative to one another, e.g. by rotating the first core member in a first direction and fixing the second core element. This causes the first and second spoke members to bend in opposite directions and preloads the spoke members. In connection with the present invention the term "preload" is understood such that the second spoke members urge into a second direction which is opposite to the first direction in which the first core element has been rotated in order to move the radially expansible element into the collapsed configuration.

By further rotating the first and second core elements relative to one another the first and second spoke members are coiled onto each other which causes a foreshortening of the spoke members. The shell elements are pulled radially towards the pair of core elements and the implant is moved into the collapsed configuration. The moving of the radially expansible element into the collapsed configuration may be supported by a crimping tool as known in the art.

Thus, starting from the collapsed configuration the radially expansible element may be gradually moved from the collapsed configuration towards the expanded configuration in a controlled manner, e.g. by rotating the first core member in the second direction and fixing the second core element. This allows a more accurate placement and repositioning of the implant in the left atrial appendage which leads to improved safety of the procedure, shorter procedure times and improved sealing rates, in particular such that small leaks below 3 mm can be closed. This may reduce the need for anticoagulation.

A controlled deployment also implies controlled fixation, so that the clinician can focus on the correct placement first and then fixate the device. The implant may be insertable into an outer sheath of a delivery catheter in the collapsed configuration of the radially expansible element and may be shifted into the expanded configuration when being positioned in the left atrial appendage. The driving and locking element for driving and locking the pair of core elements is engagable with an elongated catheter member such that the implant can be controlled by the clinician from outside of the patient's body.

In an embodiment of the invention the implant further comprises an occlusive element on a proximal portion of the radially expansible element. Preferably, the implant comprises a proximal portion and a distal portion, wherein the proximal portion faces the left atrium of the patient's heart, whereas the distal portion faces the interior of the left atrial appendage. The proximal portion of the implant may be formed by an outside surface of the implant, e.g. of the shell elements, and the occlusive element may be formed by a covering layer of material which may in part or fully cover the outside surface. Preferably, the layer covers at least the proximal portion and 10 - 100% of the flanks of the implant. The layer may cover at least the part of the surface of the implant that is in contact with blood flowing past the implant in the circulatory system. The covering layer may take the form of a membrane. It may be a layer of premade material which is added to the implant by sewing, stapling, knotting, using an adhesive or ultrasonic or thermal welding. The layer may be formed on the device directly by blow- or electrospinning, dipping or material deposition from vapor, aerosol, micro droplets, molten or otherwise liquefied material. The layer may be responsible for the hemocompatibility of the implant. The layer may be responsible for the sealing properties of the implant. The layer may serve as filtering membrane to contain thrombi and particles in the distal side of the implant. Preferably, the layer is made of a polymer, i.e. woven, nonwoven, film or membrane using materials such as polyethylene terephthalate (PET), polytetrafluoroethylene (PTFE), expanded polytetrafluoroethylene (ePTFE), polyurethane (PU), polypropylene (PP), polyvinyl alcohol (PVA), or proprietary polymers developed at Institute of Active Polymers of Helmholtz-Zentrum Hereon, i.e. poly [(L-lactide)-co-(ε-caprolactone)]/poly (D-lactide) (P(LLA-co-CL)/PDLA).

In another embodiment of the invention the occlusive element includes a porous mesh. In other words: The covering layer takes the form of a porous mesh. The mesh may be formed as a woven, braided, knitted or non-woven fabric.

In an embodiment of the invention the implant further comprises a plurality of anchor members configured to secure the implant to tissue within the left atrial appendage, wherein the anchor members are formed as spikes extending from each of the shell elements in a radial outward direction in the expanded configuration of the radially expansible element. Preferably, each shell element comprises a radial outer curved surface from which the spikes extend, the radial outer curved surface of multiple shell elements together forming the substantially circular outer circumference of the radially expansible element. Preferably, the spikes are elastic and are folded in the collapsed configuration of the expansible element such that the folded spikes abut the radial outer surface of the shell elements. The spikes may unfold vertically when the expansible element is moved into the expanded configuration. Preferably, the spikes can be folded back by driving the driving and locking element such that the first and second core elements are rotated relative to one another and the radially expansible element is moved from the expanded configuration to the collapsed configuration. In doing so the overlap of the distal ends of the shell elements may increase thereby folding the spikes to abut the radial outer surface of the shell elements. By folding the spikes the implant can be repositioned for optimal occlusion without tissue damage risk. The spikes may be reinforced with additional elements.

In another embodiment of the invention the pair of bendable spokes extends from the pair of core elements in a radial outward direction such that the first spoke member and the second spoke member when being virtually extended towards the longitudinal axis intersect the longitudinal axis. Optionally, each shell element may extend from its proximal end to its distal end at an angle relative to the longitudinal axis when projecting the longitudinal axis onto the proximal end of the corresponding shell element of between 90° to 135°, preferably between 90° to 125°, more preferably between 95° and 105°.

In another embodiment of the invention the pair of bendable spokes extends tangentially from the outer circumference of the pair of core elements in an outward direction such that the first spoke member and the second spoke member when being virtually extended towards the longitudinal axis run past the longitudinal axis. For example the first and second bendable spoke members extend tangentially in the same direction such that the first and second spoke members are positioned parallel relative to one another. Alternatively, the first spoke members and the second spoke members extend tangentially in different directions such that when looking along the longitudinal axis the first and second spoke members form an isosceles triangle, wherein the first and second spoke members form the brackets of the triangle. For example, the first spoke members extend tangentially from the first core element in a clockwise direction, whereas the second spoke members extend from the second core element in a counterclockwise direction. Optionally, each shell element may extend from its proximal end to its distal end at an angle relative to the longitudinal axis when projecting the longitudinal axis onto the proximal end of the corresponding shell element of between 90° to 135°, preferably between 90° to 125°, more preferably between 95° and 105°.

In another embodiment of the invention the implant comprises 3 to 9 pairs of bendable spokes, preferably 5 to 6 pairs of bendable spokes, more preferably 6 pairs of bendable spokes. The higher the number of spokes the more evenly the implant is pressed against the tissue of the atrial appendage. However, the higher the number of spokes the more room is required by the expansible element in the collapsed configuration leading to a higher diameter in that collapsed state. In connection with the present invention it has been found that a number of five to six pairs of bendable spoke forms is most suitable to balance an even contact on the surrounding tissue and a small size in the collapsed configuration.

In an embodiment of the invention the driving and locking element comprises a first cylindrical member adapted to be inserted into the cavity of the first core element and a second cylindrical member adapted to be inserted into the cavity of the second core element, wherein the first cylindrical member and the second cylindrical member extend along the longitudinal axis and are movable relative to one another along the longitudinal axis from a locked state, in which rotation between the first cylindrical member and the second cylindrical member is locked, to an unlocked state, in which the first and second cylindrical members are rotatable relative to one another around the longitudinal axis. The first and second cylindrical members may also be moved from the unlocked state to the locked state. By allowing the driving and locking element to be moved from an unlocked state into a locked state the rotation of the first and second core elements may be locked at any time during shifting of the radially expansible element from the collapsed configuration to the expanded configuration. Thus, when inserting the implant into the left atrial appendage the radial extension of the radially expansible element may be adapted individually to the anatomy of the patient's left atrial appendage and locked in this state of extension. Thus, the number of implant sizes the clinician has to choose from to suit the human anatomy is thereby reduced.

In another embodiment of the invention the second cylindrical member comprises a locking portion and the first cylindrical member comprises a receiving portion which is adapted to receive the locking portion of the second cylindrical member in the locked state such that the locking portion and the receiving portion form a form-looked connection. A form-locked connection is easy to manufacture and cost efficient.

In an embodiment of the invention at least one of the pair of core elements, the plurality of pairs of bendable pokes and the plurality of shell elements is made of a polymeric material. Preferably, at least one of the pair of core elements, the plurality of pairs of bendable spokes and the plurality of shell elements is made of hyperelastic polymers. Preferably, the implant is constructed of a biocompatible material and is enhanced by applying thin coating with improved hemocompatibility that will promote rapid tissue ingrowth and reduce foreign body reactions.

In another embodiment of the invention the polymeric material is chosen from the group consisting of polyisocyanurate resins (polyurethane, PU), polytetrafluoroethylene (PTFE), fluorinated ethylene propylene polymer (FEP, PTFE-FEP), polypropylene, polyglycolide (PGA), poly(glycolide-co-caprolactone) (PGCL), polylacticides (PLA, PLLA, PDLA), poly(lactide-co-glycolide) (PLGA), polycaprolacton (PCL), poly-ethylene (PE), poly-ethylene-glycol (PEG), polyethelene terephthalate (PET), polyvinyl-alcohol (PVA), poly [(L-lactide)-co-(ε-caprolactone)]/poly (D-lactide) (P(LLA-co-CL)/PDLA).

In an embodiment of the invention at least one of the pair of core elements, the plurality of pairs of bendable pokes and the plurality of shell elements is made of a metal frame. Preferably, the structural elements, i.e. shell elements, spokes and core elements can be made of a metallic material with high elasticity such as TiNi, Nitinol, in a form of a mesh of wires. The frame may be covered with a polymeric membrane. The metal frame serves as reinforcement to provide mechanical strength to the implant.

The above objects are also met by the provision of a system as defined in claim 12. Preferred embodiments are described in the appending claims.

According to the invention the system for occluding a left atrial appendage, comprises a delivery catheter including an outer sheath housing an inner elongate catheter member slidably disposed within a lumen of the outer sheath, the elongate catheter member comprising a tip and a rotatable pin mounted at the tip. The system further comprises an implant adapted to occlude the left atrial appendage comprising a radially expansible element configured to shift between a collapsed configuration and an expanded configuration. The radially expansible element comprises a pair of core elements which extends in direction of a longitudinal axis, wherein the pair of core elements comprises a first core element and a second core element, the first and second core element being stacked in direction of said longitudinal axis, the pair of core elements having an outer circumference and the first core element and the second core element each enclosing a cavity, wherein the first core element and the second core element are rotatable relative to one another.

The radially expansible element further comprises a driving and locking element arranged in the respective cavities of the pair of core elements for rotating the first core element and the second core element relative to one another so as to move the radially expansible element from the collapsed configuration into the expanded configuration and for locking the first core element and the second core element relative to one another, wherein the driving and locking element is adapted for being engaged with the rotatable pin of the elongated catheter member.

The radially expansible element further comprises a plurality of pairs of bendable spokes extending from the pair of core elements in outward direction, each of the pairs of bendable spokes having a first spoke member and a second spoke member, the first and second spoke members being stacked in direction of said longitudinal axis, and each of said first and second spoke members having an inner end and an outer end.

The radially expansible element further comprises a plurality of shell elements attached to the outer end of the spoke members, and extending transversely from the outer end of the spoke members in a curved manner, the shell elements having a proximal end and a distal end.

The inner end of the first spoke member is connected to the outer circumference of the first core element, and the inner end of the second spoke member is connected to the outer circumference of the second core element.

The outer ends of the first and second spoke members are both connected to the proximal end of the shell elements. The distal end of the shell elements is configured to overlap with a proximal end of the shell elements in the expanded configuration of the radially expansible element so as to form a substantially circular outer circumference of radially expansible element.

In an embodiment of the invention the system further comprises a manipulator adapted to rotate and retract the inner elongate catheter member relative to the outer sheath, such that upon engagement of the driving and locking element with the rotatable pin of the elongated catheter member rotation of the manipulator causes the first and second core elements to rotate relative to one another and, wherein retracting the inner elongate catheter member relative to the outer sheath causes the first and second core elements to be locked relative to one another. Thus, when inserting the implant into the left atrial appendage the radial extension of the radially expansible element may be adapted individually to the anatomy of the patient's left atrial appendage and locked in this state of extension. Thus, the number of implant sizes the clinician has to choose from to suit the human anatomy is reduced. The radially expansible element is controllable, i.e. shiftable from the collapsed configuration into the expanded configuration and vice versa, by movement of the manipulator relative to the outer sheath. This provides a more accurate placement of the implant in the left atrial appendage which leads to shorter procedure times and improved sealing rates, in particular such that small leaks below 3 mm can be closed. A controlled deployment also implies controlled fixation, so that the clinician can focus on the correct placement first and then fixate the device, e.g. by vertically unfolding the spikes to engage the surrounding tissue.

In another embodiment of the invention the driving and locking element is further adapted to unlock the first core element and the second core element in order to allow rotational movement there between and, wherein the manipulator is further adapted to advance the inner elongate catheter member relative to the outer sheath, such that upon engagement of the driving and locking element with the rotatable pin of the elongated catheter member advancing the inner elongate catheter member relative to the outer sheath causes the first and second core elements to be unlocked in order to allow rotational movement there between. By allowing the driving and locking element to be shifted from a locked state into an unlocked state the implant may be repositioned in case the implant is not properly positioned within the left atrial appendage. Once moved into the expanded configuration the expansible element may be collapsed again by rotation of the manipulator and expanded again after having been repositioned. Repositioning or retrieval of the implant does not cause potential damage to tissue or accessories.

In an embodiment of the invention the outer sheath is configured to receive the implant, wherein the radially expansible element of the implant is in the collapsed configuration. Preferably, the implant is delivered to the site of implantation percutaneously, whereby the outer sheath comprising a compressed or collapsed implant is advanced from the femoral vein into the heart and into the left atrial appendage, and unfolded at the site of implantation.

The above objects are also overcome by the provision of a method as defined in claim 16. Preferred embodiments are described in the appending claims.

According to the invention a method for occluding a left atrial appendage, comprises the following steps:
I. providing a delivery catheter including an outer sheath which houses an inner elongate catheter member slidably disposed within a lumen of the outer sheath of the delivery catheter, the elongate catheter member comprising a tip and a rotatable pin mounted at the tip, wherein an implant adapted to occlude the left atrial appendage is inserted in the outer sheath, the implant comprising a radially expansible element configured to shift between a collapsed configuration and an expanded configuration, wherein the radially expansible element comprises:
   a. a pair of core elements which extends in direction of a longitudinal axis, wherein the pair of core elements comprises a first core element and a second core element, the first and second core elements being stacked in direction of said longitudinal axis, the pair of core elements having an outer circumference and the first core element and the second core element each enclosing a cavity, wherein the first core element and the second core element are rotatable relative to one another;
   b. a driving and locking element arranged in the respective cavities of the pair of core elements for rotating the first core element and the second core element relative to one another so as to move the radially expansible element from the collapsed configuration into the expanded configuration and for locking the first core element and the second core element relative to one another, wherein the driving and locking element is engaged with the rotatable pin mounted at the tip of the elongated catheter member;
   c. a plurality of pairs of bendable spokes extending from the pair of core element in an outward direction, each of the pairs of bendable spokes having a first spoke member and a second spoke member, the first and second spoke members being stacked in direction of said longitudinal axis, and each of said first and second spoke members having an inner end and an outer end; and
   d. a plurality of shell elements attached to the outer end of the spoke members, and extending transversely from the outer end of the spoke members in a curved manner, the shell elements having a proximal end and a distal end; wherein the inner end of the first spoke member is connected to the outer circumference of the first core element, and the inner end of the second spoke member is connected to the outer circumference of the second core element;

   wherein the outer ends of the first and second spoke members are both connected to the proximal end of the shell elements; and
   wherein the distal end of the shell elements is configured to overlap with a proximal end of the shell elements in the expanded configuration of the radially expansible element so as to form a substantially circular outer circumference of the radially expansible element;
II. advancing the delivery catheter together with the implant to the left atrial appendage;
III. deploying the implant within the left atrial appendage; and
IV. releasing the implant from the delivery catheter within the left atrial appendage.

Preferably, the delivery catheter comprising a compressed or collapsed implant is advanced from the femoral vein into the heart and into the left atrial appendage, and the implant is unfolded at the site of implantation. Thereafter, the implant is released from the delivery catheterand the delivery catheter is retracted through the femoral vein.

In an embodiment of the invention deploying the implant includes:
a. pushing the implant out of the outer sheath of the delivery catheter;
b. rotating the inner elongate catheter member relative to the outer sheath thereby driving the driving and locking element and rotating the first core element relative to the second core element such that the radially expansible element is shifted from the collapsed configuration into the expanded configuration and the radially expansible element occludes the left atrial appendage;
c. retracting the inner elongate catheter member relative to the outer sheath thereby locking the driving and locking element such that the first core element and the second core element are locked relative to one another.

By rotating the inner elongate catheter memberrelative to the outer sheath the radially expansible element the clinician can control the speed of deployment and the radial expansion of the expansible element. Thus, the radially expansible element is moved from the collapsed configuration towards the expanded configuration in a controlled manner. This provides a more accurate placement of the implant in the left atrial appendage which leads to safer and shorter procedures and improved sealing rates, in particular such that small leaks below 3 mm can be closed. This may reduce the need for anticoagulation. A controlled deployment also implies controlled fixation, so that the clinician can focus on the correct placement first and then fixate the device, e.g. by vertically unfolding the spikes to engage the surrounding tissue.

Having reached the expanded configuration the driving and locking element is shifted into the locked state such that rotation of the first and second core elements relative to one another is prohibited. In case the implant is not properly positioned within the left atrial appendage the implant may be repositioned by shifting the driving and locking element back into the unlocked state. Once moved into the expanded configuration the expansible element may be collapsed again by rotating the inner elongate catheter member relative to the outer sheath of the manipulator and expanding the expansible element again after having been repositioned. Repositioning or retrieval of the implant does not cause potential damage to tissue or accessories.

In another embodiment of the invention releasing the implant includes disengaging the rotatable pin at the tip of the elongated catheter member from the driving and locking element.

### DETAILED DESCRIPTION OF THE INVENTION

The present invention is described in detail in the following with reference to the figures, in which:
- Fig. 1: shows a perspective view of an implant for occluding a left atrial appendage;
- Fig. 2: shows a perspective view of a driving and locking element;
- Fig. 3: shows a schematic view of a system for occluding a left atrial appendage;
- Figs. 4a to 4f: show multiple steps of the radially expansible element of the present invention moving from the collapsed configuration to the expanded configuration;
- Fig. 5: shows a perspective view of a second embodiment of an implant for occluding a left atrial appendage;
- Fig. 6: shows a perspective view of a third embodiment of an implant for occluding a left atrial appendage;
- Fig. 7: shows a perspective view of a fourth embodiment of an implant for occluding a left atrial appendage;
- Fig. 8: shows a perspective view of a fifth embodiment of an implant for occluding a left atrial appendage and
- Fig. 9: shows a perspective view of a sixth embodiment of an implant for occluding a left atrial appendage.

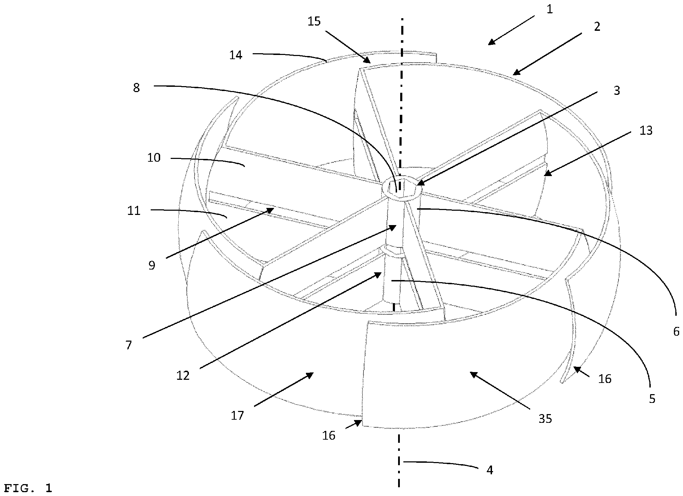

Fig. 1 shows an implant 1 for occluding a left atrial appendage comprising a radially expansible element 2 configured to shift between a collapsed configuration and an expanded configuration. The radially expansible element 2 comprises a pair of core elements 3 which extends in direction of a longitudinal axis 4. The pair of core elements 3 comprises a first core element 5 and a second core element 6. The first and second core elements 5, 6 are stacked in direction of the longitudinal axis 4. The pair of core elements 3 has an outer circumference 7 and the first core element 5 and the second core element 6 each enclose a cavity 8. The first core element 5 and the second core element 6 are rotatable relative to one another.

The implant 1 further comprises six pairs of bendable spokes 9. Each of the pairs of bendable spokes 9 has a first spoke member 10 and a second spoke member 11. The pairs of bendable spokes 9 extend from the pair of core elements 3 in a radial outward direction such that the first spoke member 10 and the second spoke member 11 when being virtually extended towards the longitudinal axis 4 intersect the longitudinal axis 4. The first and second spoke members 10, 11 are stacked in direction of the longitudinal axis 4. Each of the first and second spoke members 10, 11 has an inner end 12 and an outer end 13.

The implant 1 further comprises a plurality of shell elements 14 attached to the outer end 13 of the spoke members 11, 12 and extending transversely from the outer end 13 of the spoke members 11, 12 in a curved manner. The shell elements 14 have a proximal end 15, a distal end 16 and a radial outer curved surface 35.

The inner end 12 of the first spoke member 10 is connected to the outer circumference 7 of the first core element 5. The inner end 12 of the second spoke member 11 is connected to the outer circumference 7 of the second core element 6. The outer ends 13 of the first and second spoke members 10, 11 are both connected to the proximal end 15 of the shell elements 14.

The distal end 16 of the shell elements 14 is configured to overlap with a proximal end 15 of the shell elements 14 in the expanded configuration of the radially expansible element 2 so as to form a substantially circular outer circumference 17 of the radially expansible element 2.

The pair of core elements 3, the plurality of pairs of bendable spokes 9 and the plurality of shell elements 14 are made of a polymeric material. The polymeric material is chosen from the group consisting of polyisocyanurate resins (polyurethane, PU), polytetrafluoroethylene (PTFE), fluorinated ethylene propylene polymer (FEP, PTFE-FEP), polypropylene, polyglycolide (PGA), poly(glycolide-co-caprolactone) (PGCL), polylacticides (PLA, PLLA, PDLA), poly(lactide-co-glycolide) (PLGA), polycaprolacton (PCL), poly-ethylene (PE), poly-ethylene-glycol (PEG), polyethelene terephthalate (PET) and polyvinyl-alcohol (PVA).

Optionally, the implant 1 further comprises an occlusive element (not shown) on a proximal portion 18 of the radially expansible element 2, the occlusive element including a porous mesh.

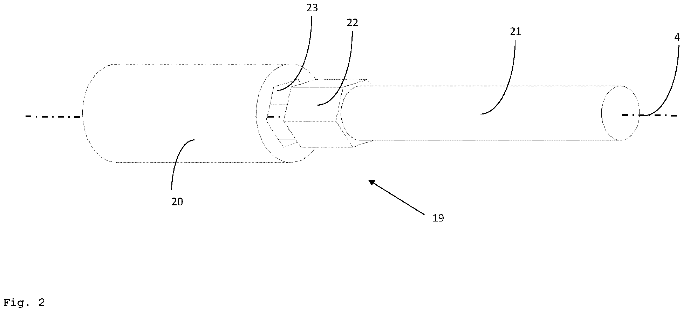

Fig. 2 shows a driving and locking element 19 which is arranged in the respective cavities 8 of the pair of core elements 3 (Fig. 1). The driving and locking element 19 is configured to rotate the first core element 5 and the second core element 6 relative to one another so as to move the radially expansible element 2 from the collapsed configuration into the expanded configuration and to lock the first core element 5 and the second core element 6 relative to one another. The driving and locking element 19 is further adapted to unlock the first core element 5 and the second core element 6 in order to allow rotational movement there between. The driving and locking element 19 is engagable with a rotatable pin mounted at a tip of an elongated catheter member (not shown).

The driving and locking element 19 comprises a first cylindrical member 20 adapted to be inserted into the cavity 8 of the first core element 5 and a second cylindrical member 21 adapted to be inserted into the cavity 8 of the second core element 6. The first cylindrical member 20 and the second cylindrical member 21 extend along the longitudinal axis 4 and are movable relative to one another along the longitudinal axis 4 from a locked state, in which rotation between the first cylindrical member 20 and the second cylindrical member 21 is locked, to an unlocked state, in which the first and second cylindrical members 20, 21 are rotatable relative to one another around the longitudinal axis 4. The second cylindrical member 21 comprises a locking portion 22 and the first cylindrical member 20 comprises a receiving portion 23 which is adapted to receive the locking portion 22 of the second cylindrical member 21 in the locked state such that the locking portion 22 and the receiving portion 23 form a form-looked connection.

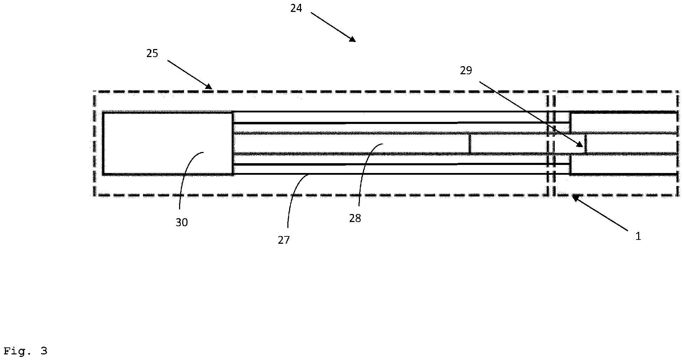

Fig. 3 shows a system 24 for occluding a left atrial appendage, comprising a delivery catheter 25 including an outer sheath 27 which houses an inner elongate catheter member 28 slidably disposed within a lumen of the outer sheath 27. The elongate catheter member 28 comprises a tip 29 and a rotatable pin (no shown) mounted at the tip 29.

The system 24 further comprises the implant 1 as shown in Figs. 1 and 2 which is adapted to occlude the left atrial appendage. The implant 1 is inserted into the outer sheath 27, wherein the radially expansible element 2 of the implant 1 is in the collapsed configuration. The driving and locking element 19 of the implant 1 is engaged with the pin arranged on the tip 29 of the elongate catheter member 28.

The system 24 further comprises a manipulator 30 adapted to rotate and retract the inner elongate catheter member 28 relative to the outer sheath 27. Rotation of the manipulator 30 causes the first and second core elements 5, 6 (Fig. 1) to rotate relative to one another. Retracting the inner elongate catheter member 28 relative to the outer sheath 27 causes the first and second core elements 5, 6 to be locked relative to one another. The manipulator 30 is further adapted to advance the inner elongate catheter member 28 relative to the outer sheath 27, such that advancing the inner elongate catheter member 28 relative to the outer sheath 27 causes the first and second core elements 5, 6 to be unlocked in order to allow rotational movement there between.

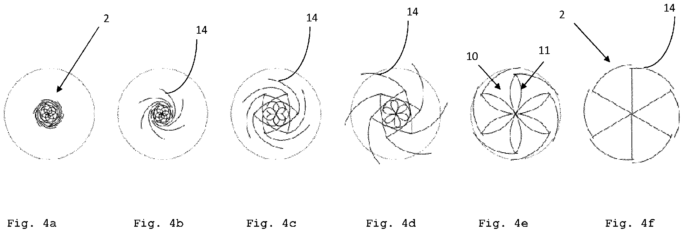

Figs. 4a to 4f show multiple steps of the radially expansible element 2 moving from the collapsed configuration (shown in Fig. 4a) to the expanded configuration (shown in Fig. 4f). The expansible element 2 may be brought in the collapsed configuration as shown in Fig. 4a by rotating the first and second core elements 5, 6 relative to one another, e.g. by rotating the first core member 5 in a first direction and fixing the second core element 6. This causes the first and second spoke members 10, 11 to bend in opposite directions (see Figure 4e). By further rotating the first and second core elements 5, 6 relative to one another the first and second spoke members 10, 11 are coiled onto each other which causes a foreshortening of the spoke members 10, 11. The shell elements 14 are pulled radially towards the pair of core elements 3 and the implant 1 collapses.

The moving of the implant 1 into the collapsed configuration may be supported by a crimping tool (not shown) as known in the art. However, in order to be able to gradually extend the radially expansible element 2 from the collapsed configuration to the expanded configuration as will be described in the following the first and second spoke members 10, 11 need to be preloaded. Preloading the spoke members 10, 11 is achieved by rotation of the first and second core elements 5, 6 relative to one another as described above such that the first and second spoke members 10, 11 are bent in opposite directions (see Figure 4e).

Assuming that the first and second spoke members are preloaded and the radially expansible element 2 is in the collapsed configuration the radially expansible element 2 is moved into the expanded configuration as follows:

Due to the preload of the first and second spoke members 10, 11 the first and second core elements 5, 6 urge to rotate relative to each other, e.g. when the second core member 6 is fixed the first core member 5 urges to rotate into a second direction which is opposite to the first direction as defined above with regard to the moving of the radically expansible element 2 from the expanded configuration into the collapsed configuration.

By turning the manipulator 30 relative to the outer sheath 27, e.g. by rotating the manipulator 30 in the second direction and fixing the outer sheath 27 the first and second core elements 5, 6 (Fig. 1) rotate relative to one another thereby moving the radially expansible element 2 from the collapsed configuration into the expanded configuration as shown in Fig. 4f. By rotating the first and second core elements 5, 6 in opposite directions the first and second spoke members 10, 11 are uncoiled from the first and second core elements 5, 6 causing a radial extension so as to push the shell elements 14 radially outward. At any time in between the collapsed configuration and the expanded configuration the inner elongate catheter member 28 may be retracted relative to the outer sheath 27 by use of the manipulator 30 causing the first and second core elements 5, 6 to be locked relative to one another.

In the following a method for occluding a left atrial appendage is described with reference to Figure 1 to 4f.

In a first step the delivery catheter 25 as described above with reference to Fig. 3 is provided, wherein the implant 1 which is adapted to occlude the left atrial appendage is inserted in the outer sheath 27.

In a second step the delivery catheter 25 is advanced together with the implant 1 to the left atrial appendage. Preferably, the outer sheath 27 comprising the compressed or collapsed implant 1 is advanced from the femoral vein into the heart and into the left atrial appendage of the patient (not shown).

Thereafter, the implant 1 is pushed out of the outer sheath 27 and positioned in the left atrial appendage.

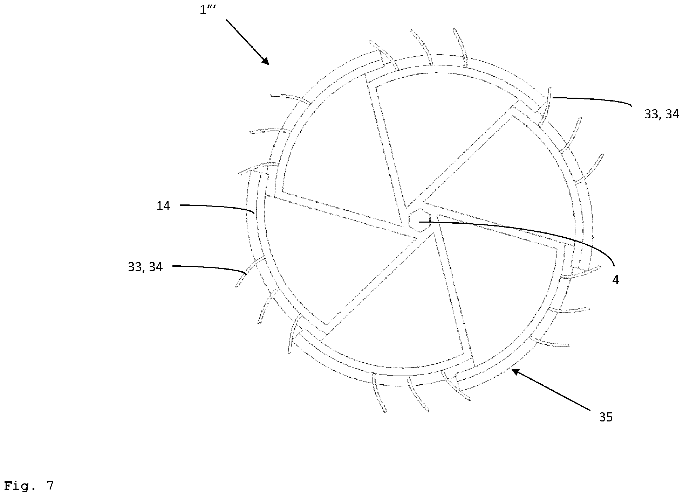

Subsequently, the inner elongate catheter member 28 is rotated relative to the outer sheath 27 thereby driving the driving and locking element 19 and rotating the first core element 5 relative to the second core element 6 such that the radially expansible element 2 is shifted from the collapsed configuration into the expanded configuration and the radially expansible element 2 occludes the left atrial appendage. In case the implant 1 comprises anchor members 33, which will be explained later on in detail with regard to Fig. 7, the anchor members engage the tissue surrounding the left artial appendage and the implant 1 is fixed.

In case the implant 1 is not properly positioned within the left atrial appendage the inner elongate catheter member 28 is rotated in an opposite direction relative to the outer sheath 27 thereby driving the driving and locking element 19 and rotating the first core element 5 relative to the second core element 6 such that the radially expansible element 2 is shifted from the expanded configuration into the collapsed configuration. After repositioning the implant 1 the implant 1 is again moved to the expanded configuration as described above and fixed in the left atrial appendage.

In a subsequent step, the inner elongate catheter member 28 is retracted relative to the outer sheath 27 thereby locking the driving and locking element 19 such that the first core element 5 and the second core element 6 are locked relative to one another.

Thereafter, the rotatable pin at the tip 29 of the inner elongated catheter member 28 is disengaged from the driving and locking element 19, thereby releasing the implant 1 from the delivery catheter 25 within the left atrial appendage.

Finally, the delivery catheter 25is retracted from the patient's body, preferably through the femoral vein.

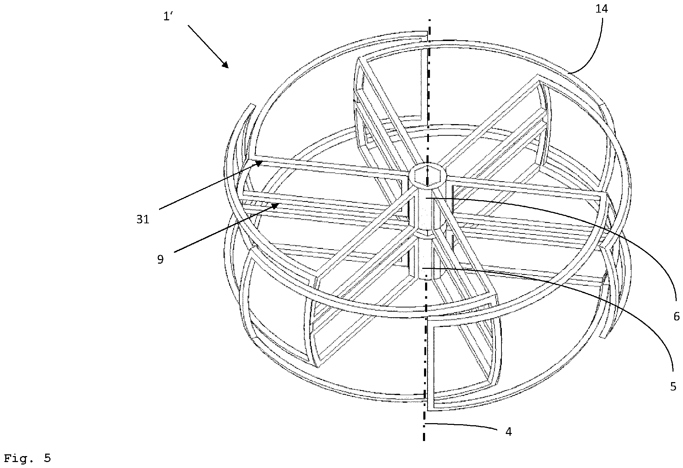

Fig. 5 shows a second embodiment of the implant 1 for occluding a left atrial appendage. The implant 1' differs from the one shown in Fig. 1 in that the pair of core elements 5, 6, the plurality of pairs of bendable pokes 9 and the plurality of shell elements 14 is made of a metal frame 31. The metal frame 31 is made of Nitinol. The metal frame 31 is covered by polymeric membrane 32. Preferably, the polymeric membrane 32 is made of polyethylene terephthalate (PET), polytetrafluoroethylene (PTFE), expanded polytetrafluoroethylene (ePTFE), polyurethane (PU), polypropylene (PP), polyvinyl alcohol (PVA), or proprietary polymers developed at Institute of Active Polymers of Helmholtz-Zentrum Hereon, i.e. poly [(L-lactide)-co-(ε-caprolactone)]/poly (D-lactide) (P(LLA-co-CL)/PDLA).

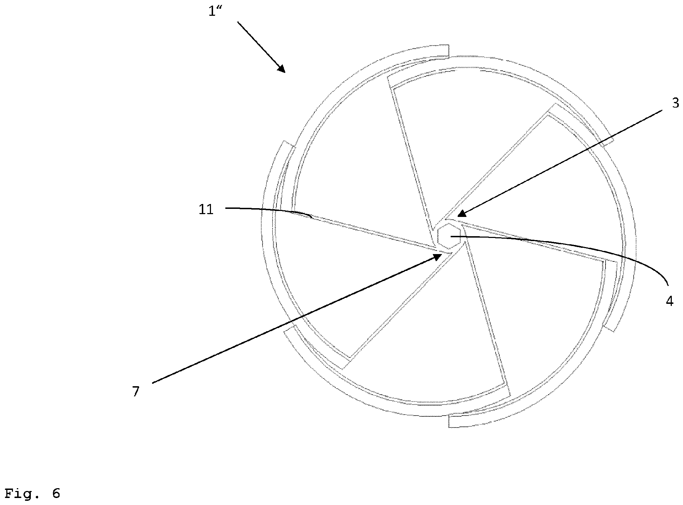

Fig. 6 shows a third embodiment of the implant 1 for occluding a left atrial appendage. The implant 1" differs from the one shown in Fig. 1 in that 1 the pair of bendable spokes 9 extends tangentially from the outer circumference 7 of the pair of core elements 3 in an outward direction such that the first spoke member 10 and the second spoke 11 member when being virtually extended towards the longitudinal axis 4 run past the longitudinal axis 4. As shown in Fig. 6 the first and second bendable spoke members 10, 11 extend tangentially in the same direction such that the first and second spoke members 10, 11 are positioned parallel relative to one another. The first and second spoke members 10, 11 extend tangentially from the first and second core element 5, 6 in a clockwise direction.

Fig. 7 shows a fourth embodiment of the implant 1 for occluding a left atrial appendage. The implant 1‴ differs from the one as shown in Fig. 6 in that it further comprises a plurality of anchor members 33 configured to secure the implant 1‴ to tissue within the left atrial appendage. The anchor members 33 are formed as spikes 34 extending from each of the shell elements 14 in a radial outward direction in the expanded configuration of the radially expansible element 1‴. The spikes 34 are elastic and are folded in the collapsed configuration of the expansible element 2 such that the folded spikes 34 abut the radial outer surface 35 of the shell elements 14. The spikes 34 unfold vertically when the expansible element 2 is moved into the expanded configuration. The spikes 34 are not limited to the third embodiment of the implant 1". The anchor elements 33 may be applied to any embodiment of the implant 1 as described herein.

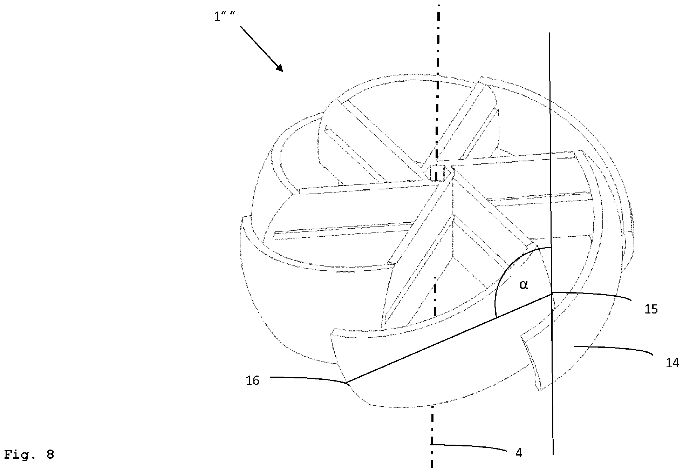

Fig. 8 shows a fifth embodiment of an implant 1 for occluding a left atrial appendage. The implant 1ʺʺ differs from the one as shown in Fig. 6 in that each of the shell elements 14 extends from its proximal end 15 to its distal end 16 at an angle α relative to the longitudinal axis 4 when projecting the longitudinal axis 4 onto the proximal end 15 of the corresponding shell element 14 of between 90° to 135°, preferably between 90° to 125, more preferably between 95° and 105°. The extension of the shell elements 14 at an angle relative to the longitudinal axis 4 as described above is not limited to the third embodiment of the implant 1". The extension of the shell elements 14 at an angle relative to the longitudinal axis 4 may be combined with any embodiment of the implant 1 as described herein.

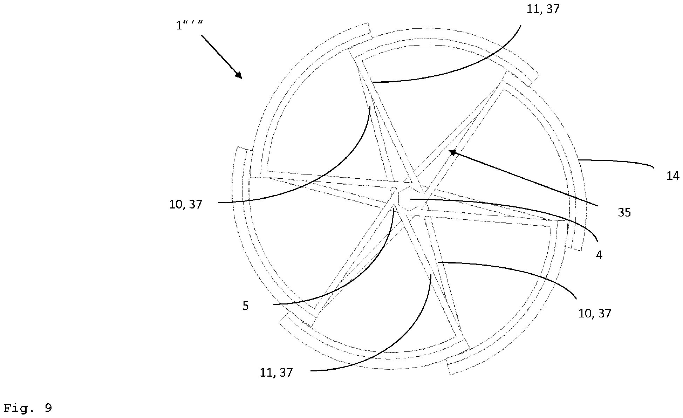

Fig. 9 shows a sixth embodiment of the implant 1 for occluding a left atrial appendage. The implant 1‴ʺ differs from the one shown in Fig. 6 in that the first spoke members 10 and the second spoke members 11 extend tangentially from the first and second core elements 5, 6 in different directions. When looking along the longitudinal axis 4 the first and second spoke members 10, 11 form an isosceles triangle 36, wherein the first and second spoke members 10, 11 form the brackets 37 of the triangle. The first spoke members 10 extend tangentially from the first core element 5 in a clockwise direction, whereas the second spoke members 11 extend from the second core element 6 in a counterclockwise direction.

### Reference numerals

- 1, 1', 1", 1‴, 1‴ʺ: Implant
- 2: Expansible element
- 3: Pair of core elements
- 4: Longitudinal axis
- 5: First core element
- 6: Second core element
- 7: Outer circumference (pair of core elements)
- 8: Cavity (first and second core elements)
- 9: Pair of bendable spokes
- 10: first spoke member
- 11: second spoke member
- 12: inner end (spoke members)
- 13: outer end (spoke members)
- 14: shell element
- 15: proximal end (shell elements)
- 16: distal end (shell elements)
- 17: circular outer circumference (Expansible element)
- 18: proximal portion (expansible element)
- 19: driving and locking element
- 20: first cylindrical member (driving and locking element)
- 21: second cylindrical member (driving and locking element)
- 22: locking portion (driving and locking element)
- 23: receiving portion (driving and locking element)
- 24: system
- 25: delivery catheter
- 27: outer sheath
- 28: elongate catheter member
- 29: tip (catheter member)
- 30: manipulator (delivery catheter)
- 31: metal frame
- 32: polymeric membrane
- 33: anchor members
- 34: spikes
- 35: outer surface (shell element)
- α: angle
- 36: isosceles triangle
- 37: brackets (triangle)

## Claims

1. An implant for occluding a left atrial appendage comprising a radially expansible element (2) configured to shift between a collapsed configuration and an expanded configuration, wherein the radially expansible element (2) comprises:
a. a pair of core elements (3) which extends in direction of a longitudinal axis (4), wherein the pair of core elements (3) comprises a first core element (5) and a second core element (6), the first and second core elements (5, 6) being stacked in direction of said longitudinal axis (4),
the pair of core elements (3) having an outer circumference (7) and the first core element (5) and the second core element (6) each enclosing a cavity (8), wherein the first core element (5) and the second core element (6) are rotatable relative to one another;
b. a driving and locking element (19) arranged in the respective cavities (8) of the pair of core elements (3) for rotating the first core element (5) and the second core element (6) relative to one another so as to move the radially expansible element (2) from the collapsed configuration into the expanded configuration and for locking the first core element (5) and the second core element (6) relative to one another, wherein the driving and locking element is (19) adapted for being engaged with a rotatable pin mounted at the tip (29) of an elongated catheter member (28);
c. a plurality of pairs of bendable spokes (9) extending from the pair of core elements (3) in an outward direction, each of the pairs of bendable spokes (9) having a first spoke member (10) and a second spoke member (11), the first and second spoke members (10, 11) being stacked in direction of said longitudinal axis (4), and each of said first and second spoke members (10, 11) having an inner end (12) and an outer end (13); and
d. a plurality of shell elements (14) attached to the outer end (13) of the spoke members (10, 11), and extending transversely from the outer end (13) of the spoke members (10, 11) in a curved manner, the shell elements (14) having a proximal end (15) and a distal end (16);
wherein the inner end (12) of the first spoke member (10) is connected to the outer circumference (7) of the first core element (5), and the inner end (12) of the second spoke (11) member is connected to the outer circumference (7) of the second core element (6);
wherein the outer ends (13) of the first and second spoke members (10, 11) are both connected to the proximal end (15) of the shell elements (14); and
wherein the distal end (16) of the shell elements (14) is configured to overlap with a proximal end (15) of the shell elements (14) in the expanded configuration of the radially expansible element (2) so as to form a substantially circular outer circumference (17) of the radially expansible element (2).

2. The implant of claim 1 further comprising an occlusive element on a proximal portion (18) of the radially expansible element (2).

3. The implant of claim 2, wherein the occlusive element includes a porous mesh.

4. The implant of any one of the preceding claims further comprising a plurality of anchor members (33) configured to secure the implant (1, 1', 1", 1‴) to tissue within the left atrial appendage, wherein the anchor members (33) are formed as spikes (34) extending from each of the shell elements (14) in a radial outward direction in the expanded configuration of the radially expansible element (2).

5. The implant of any one of the preceding claims, wherein the pair of bendable spokes (9) extends from the pair of core elements (3) in a radial outward direction such that the first spoke member (10) and the second spoke member (11) when being virtually extended towards the longitudinal axis (4) intersect the longitudinal axis (4).

6. The implant of any one of claims 1 to 4, wherein the pair of bendable spokes (9) extends tangentially from the outer circumference (7) of the pair of core elements (3) in an outward direction such that the first spoke member (10) and the second spoke member (11) when being virtually extended towards the longitudinal axis (4) run past the longitudinal axis (4).

7. The implant of any one of the preceding claims, wherein the implant comprises 3 to 9 pairs of bendable spokes (9), preferably 5 to 6 pairs of bendable spokes(9), more preferably 6 pairs of bendable spokes(9).

8. The implant of any one of the preceding claims, wherein the driving and locking element (19) comprises a first cylindrical member (20) adapted to be inserted into the cavity (8) of the first core element (5) and a second cylindrical member (21) adapted to be inserted into the cavity (8) of the second core element (6), wherein the first cylindrical member (20) and the second cylindrical member (21) extend along the longitudinal axis (4) and are movable relative to one another along the longitudinal axis (4) from a locked state, in which rotation between the first cylindrical member (20) and the second cylindrical member (21) is locked, to an unlocked state, in which the first and second cylindrical members (20, 21) are rotatable relative to one another around the longitudinal axis (4).

9. The implant of claims 8, wherein the second cylindrical member (21) comprises a locking portion (22) and the first cylindrical member (20) comprises a receiving portion (23) which is adapted to receive the locking portion (22) of the second cylindrical member (21) in the locked state such that the locking portion (22) and the receiving portion (23) form a form-looked connection.

10. The implant of any one of the preceding claims, wherein at least one of the pair of core elements (3), the plurality of pairs of bendable spokes (9) and the plurality of shell elements (14) is made of a polymeric material.

11. The implant of claim 10, wherein the polymeric material is chosen from the group consisting of polyisocyanurate resins (polyurethane, PU), polytetrafluoroethylene (PTFE), fluorinated ethylene propylene polymer (FEP, PTFE-FEP), polypropylene, polyglycolide (PGA), poly(glycolide-co-caprolactone) (PGCL), polylacticides (PLA, PLLA, PDLA), poly(lactide-co-glycolide) (PLGA), polycaprolacton (PCL), poly-ethylene (PE), poly-ethylene-glycol (PEG), polyethelene terephthalate (PET), polyvinyl-alcohol (PVA) and poly [(L-lactide)-co-(ε-caprolactone)]/poly (D-lactide) (P(LLA-co-CL)/PDLA).

12. The implant of any one of the preceding claims, wherein at least one of the pair of core elements (3), the plurality of pairs of bendable pokes (9) and the plurality of shell elements (14) is made of a metal frame.

13. A system for occluding a left atrial appendage, comprising:
i. a delivery catheter (25) including an outer sheath (27) which houses an inner elongate catheter member (28) slidably disposed within a lumen of the outer sheath (27), the elongate catheter member (28) comprising a tip (29) and a rotatable pin mounted at the tip (29); and
ii. an implant (1, 1', 1", 1‴) adapted to occlude the left atrial appendage comprising a radially expansible element (2) configured to shift between a collapsed configuration and an expanded configuration, wherein the radially expansible element (2) comprises:
a. a pair of core elements (3) which extends in direction of a longitudinal axis (4), wherein the pair of core elements (3) comprises a first core element (5) and a second core element (6), the first and second core element (5, 6) being stacked in direction of said longitudinal axis (4),
the pair of core elements (3) having an outer circumference (7) and the first core element (5) and the second core element (6) each enclosing a cavity (8), wherein the first core element (5) and the second core element (6) are rotatable relative to one another;
b. a driving and locking element (19) arranged in the respective cavities (8) of the pair of core elements (3) for rotating the first core element (5) and the second core element (6) relative to one another so as to move the radially expansible element (2) from the collapsed configuration into the expanded configuration and for locking the first core element (5) and the second core element (6) relative to one another, wherein the driving and locking element (19) is adapted for being engaged with the rotatable pin of the elongated catheter member(28);
c. a plurality of pairs of bendable spokes (9) extending from the pair of core elements (3) in outward direction, each of the pairs of bendable spokes (9) having a first spoke member (10) and a second spoke member (11), the first and second spoke members (10, 11) being stacked in direction of said longitudinal axis (4), and each of said first and second spoke members (10, 11) having an inner end (12) and an outer end (13); and
d. a plurality of shell elements (14) attached to the outer end (13) of the spoke members (10, 11), and extending transversely from the outer end (13) of the spoke members (10, 11) in a curved manner, the shell elements (14) having a proximal end (15) and a distal end (16);
wherein the inner end (12) of the first spoke member (10) is connected to the outer circumference (7) of the first core element (5), and the inner end (12) of the second spoke member (11) is connected to the outer circumference (7) of the second core element (6);
wherein the outer ends (13) of the first and second spoke members (10, 11) are both connected to the proximal end (15) of the shell elements (14); and
wherein the distal end (16) of the shell elements (14) is configured to overlap with a proximal end (15) of the shell elements (14) in the expanded configuration of the radially expansible element (2) so as to form a substantially circular outer circumference (17) of the radially expansible element (2).

14. The system of claim 13 the system further comprising a manipulator (30) adapted to rotate and retract the inner elongate catheter member (28) relative to the outer sheath (27), such that upon engagement of the driving and locking element (19) with the rotatable pin of the elongated catheter member (28) rotation of the manipulator (30) causes the first and second core elements (5, 6) to rotate relative to one another and, wherein retracting the inner elongate catheter member (28) relative to the outer sheath (27) causes the first and second core elements (5, 6) to be locked relative to one another.

15. The system of claim 14 wherein the driving and locking element (19) is further adapted to unlock the first core element (5) and the second core element (6) in order to allow rotational movement there between and, wherein the manipulator (30) is further adapted to advance the inner elongate catheter member (28) relative to the outer sheath (27), such that upon engagement of the driving and locking element (19) with the rotatable pin of the elongated catheter member (28) advancing the inner elongate catheter member (28) relative to the outer sheath (27) causes the first and second core elements (5, 6) to be unlocked in order to allow rotational movement there between.

16. The system of any one of claims 13 to 15 wherein the outer sheath (27) is configured to receive the implant (1, 1', 1", 1‴), wherein the radially expansible element (2) of the implant (1, 1', 1", 1‴) is in the collapsed configuration.

17. A method for occluding a left atrial appendage, comprising:
i. providing a delivery catheter (25) including an outer sheath (27) which houses an inner elongate catheter member (28) slidably disposed within a lumen of the outer sheath (27), the elongate catheter member (28) comprising a tip (29) and a rotatable pin mounted at the tip (29),
wherein an implant (1, 1', 1", 1‴) adapted to occlude the left atrial appendage is inserted in the outer sheath (27), the implant (1, 1', 1", 1‴) comprising a radially expansible element (2) configured to shift between a collapsed configuration and an expanded configuration, wherein the radially expansible element (2) comprises:
a. a pair of core elements (3) which extends in direction of a longitudinal axis (4), wherein the pair of core elements (3) comprises a first core element (5) and a second core element (6), the first and second core elements (5, 6) being stacked in direction of said longitudinal axis (4),
the pair of core elements (3) having an outer circumference (7) and the first core element (5) and the second core element (6) each enclosing a cavity (8), wherein the first core element (5) and the second core element (6) are rotatable relative to one another;
b. a driving and locking element (19) arranged in the respective cavities (8) of the pair of core elements (3) for rotating the first core element (5) and the second core element (6) relative to one another so as to move the radially expansible element (2) from the collapsed configuration into the expanded configuration and for locking the first core element (5) and the second core element (6) relative to one another, wherein the driving and locking element (19) is engaged with the rotatable pin mounted at the tip (29) of the elongated catheter member (28);
c. a plurality of pairs of bendable spokes (9) extending from the pair of core element (3) in an outward direction, each of the pairs of bendable spokes (9) having a first spoke member (10) and a second spoke member (11), the first and second spoke members (10, 11) being stacked in direction of said longitudinal axis (4), and each of said first and second spoke members (10, 11) having an inner end (12) and an outer end (13); and
d. a plurality of shell elements (14) attached to the outer end (13) of the spoke members (9), and extending transversely from the outer end (13) of the spoke members (10, 11) in a curved manner, the shell elements (14) having a proximal end (15) and a distal end (16);
wherein the inner end (12) of the first spoke member (10) is connected to the outer circumference (7) of the first core element (5), and the inner end (12) of the second spoke member (11) is connected to the outer circumference (7) of the second core element (6);
wherein the outer ends (13) of the first and second spoke members (10, 11) are both connected to the proximal end (15) of the shell elements (14); and wherein the distal end (16) of the shell elements (14) is configured to overlap with a proximal end (15) of the shell elements (14) in the expanded configuration of the radially expansible element (2) so as to form a substantially circular outer circumference (17) of the radially expansible element (2);
ii. advancing the delivery catheter (25) together with the implant (1, 1', 1", 1‴) to the left atrial appendage;
iii. deploying the implant (1, 1', 1", 1‴) within the left atrial appendage; and
iv. releasing the implant (1, 1', 1", 1‴) from the delivery catheter (25) within the left atrial appendage.

18. The method of claim 17, wherein deploying the implant (1, 1', 1", 1‴) includes:
a. pushing the implant (1, 1', 1", 1‴) out of the outer sheath (27);
b. rotating the inner elongate catheter member (28) relative to the outer sheath (27) thereby driving the driving and locking element (19) and rotating the first core element (5) relative to the second core element (6) such that the radially expansible element (2) is shifted from the collapsed configuration into the expanded configuration and the radially expansible element (2) occludes the left atrial appendage;
c. retracting the inner elongate catheter member (28) relative to the outer sheath (27) thereby locking the driving and locking element (19) such that the first core element (5) and the second core element (6) are locked relative to one another.

19. The method of any one of claims 17 and 18, wherein releasing the implant (1, 1', 1", 1‴) includes disengaging the rotatable pin at the tip (29) of the elongated catheter member (28) from the driving and locking element (19).
